Europäisches Patentamt

(19) European Patent Office

Office européen des brevets

(11) Publication number: 0 063 572
B1

(12) EUROPEAN PATENT SPECIFICATION

(45) Date of publication of patent specification: 05.11.86

(21) Application number: 81902795.4

(22) Date of filing: 16.10.81

(86) International application number:
PCT/AU81/00145

(87) International publication number:
WO 82/01483 13.05.82 Gazette 82/12

(51) Int. Cl.⁴: **B 09 B 3/00,** A 61 L 11/00,
A 23 K 1/08, A 23 K 1/10,
A 23 K 1/14, B 03 B 9/06,
C 12 P 7/08, C 12 P 5/02

(54) PROCESS FOR SEPARATING AND RECOVERING OPRGANIC AND INORGANIC MATTER FROM WASTE MATERIAL.

(30) Priority: 03.11.80 AU 6331/80

(43) Date of publication of application:
03.11.82 Bulletin 82/44

(45) Publication of the grant of the patent:
05.11.86 Bulletin 86/45

(84) Designated Contracting States:
AT CH DE FR GB LI NL SE

(56) References cited:
EP-A-0 005 703
AU-A- 55 298
AU-A- 85 678
AU-B- 29 770
AU-B- 64 534
CH-A- 534 010
FR-A- 738 578
FR-A- 912 877
US-A-3 787 583
US-A-3 870 798
US-A-4 050 899

(73) Proprietor: HOLLOWAY, Clifford Carlton
Unit 1 19 The Esplanade
South Perth, W.A. 6151 (AU)

(72) Inventor: HOLLOWAY, Clifford Carlton
Unit 1 19 The Esplanade
South Perth, W.A. 6151 (AU)

(74) Representative: Carpmael, John William
Maurice et al
CARPMAELS & RANSFORD 43 Bloomsbury
Square
London, WC1A 2RA (GB)

Courier Press, Leamington Spa, England.

## Description

This invention relates to a process for treating waste material and more particularly to a process for treating waste material such as commercial, industrial, agricultural, household and restaurant waste for the recovery of useful organic and inorganic matter contained therein.

In one particularly important aspect, the invention relates to the treatment of municipal solid waste and to the processing of swill and household garbage and sewage. The term "swill" is used throughout this specification to refer to all putrescible organic municipal and agricultural waste. Such waste includes waste food discarded by households and, particularly, hotels, restaurants and other commercial catering establishments including hospitals, and thus can be of a high nutritional value.

Heretofore, various methods have been devised for the treatment and disposal of waste material including landfill, ocean dumping, composting, heat treatment and incineration. With the tremendous increase in volume of waste produced, such methods have become environmentally unsafe, inefficient and very expensive. Also, swill from hotels, restaurants and other commercial catering establishments has in the past been fed to animals as a method of disposal, but in recent years this has been discouraged due to the fear of introducing disease to such animals.

Furthermore, conventional recycling processes used in cooperation with such disposal methods only separate out the solids from the remainder of the waste and reduce the size thereof for the recovery of ferrous metals and other useful solids such as glass and the like contained therein. This may be accomplished by subjecting the solids to magnets for the recovery of ferrous metals or by subjecting them to crushers and hammer mills for size reduction. Also, the solids may be passed through fluids moving at various speeds whereby they are separated and classified according to density and aerodynamic qualities. Such conventional recovery processes have only achieved limited success due to the fact that they recover only limited amounts of reusable material with waste material still remaining for disposal.

In addition, a result of the bans and other measures taken to discourage the feeding of swill to animals has been that the use of commercial and domestic garbage grinders for the disposal of food wastes has increased. Thus, garbage grinders are now in use in many household kitchen sinks and, with the advent of commercial garbage grinders in hospitals, hotels, supermarkets, and so on, the food scraps from commercial institutions which were previously collected and fed to animals as swill are now diverted into the sewage system. This diversion of the swill into the sewage system, whilst avoiding problems which would arise if it was fed to animals, gives rise to problems in itself as it causes a further load on what is often an already over-loaded sewage system.

A process and apparatus for fragmenting certain solid wastes is disclosed in US—A—4 050 899 (Grube et al.). This process comprises according to the preamble of claim 1 the steps of:

(a) feeding said waste material feedstock into a closed chamber;

(b) subjecting said waste material feedstock in said chamber to heat under a pressure ranging preferably from 1.38 to 6.89 bar (20 to 100 psi) for a period ranging preferably from twenty minutes to two hours to sterilize said waste material feedstock and soften said organic matter contained therein;

(c) releasing the pressure in said chamber suddenly; and

(d) subjecting the organic component of said sterilized feedstock to fermentation to form a fermentation mash.

In accordance with the present invention, there is provided a process for treating waste material for the recovery of organic and inorganic matter which is safe and more reliable than the methods mentioned above. The improved process is simple and economical of operation and recovers more useful material than the prior conventional methods. The process will therefore significantly reduce the overload of waste material going to landfills and sewage plants by recovering not only solid inorganic matter but organic matter as well.

It is an object of the invention to provide an efficient waste recovery process which is adapted to treat various types of waste material, including materials such as paper, plastic, metal, glass, waste food and wood chips, for the recovery of useful organic and inorganic matter, including animal foodstuffs and fuel materials.

According to the present invention there is provided a process for treating a waste material feedstock for the separation and recovery of organic and inorganic matter contained therein, which comprises the steps (a) to (d) described above and is characterised in that:

(e) said waste material feedstock is fed into a container having perforations therein mounted within said closed chamber in spaced relation to the inner surface of said closed chamber;

(f) said waste material feedstock is agitated while in said container before said sudden release of pressure;

(g) during said sudden release of pressure, the softened organic matter is forced outwardly of said container through said perforations, so that a softened essentially organic component of the sterilized feedstock is separated from solid inorganic matter in said container; and

(h) said separated softened essentially organic component is then subjected to said fermentation step.

In carrying out the present invention, separation of the solid inorganic materials from the feedstock and subjecting the organic component of the feedstock to heat and/or pressure is conveniently carried out in a single step. This is carried out by feeding the waste material feedstock into a container having perforations therein and mounted within a closed chamber in spaced relation to the inner surface of said closed chamber. On a small scale, this may be carried out in an autoclave. However, large scale equipment designed on similar principles can also be used. After introduction of the feedstock, it is agitated while in the container and subjected to heat and/or pressure, for example by introduction of steam to the closed chamber, at a pressure from 1.38 to 6.89 bar, preferably 4.14 bar (20 to 100 psi, preferably 60 psi) for a period from 20 minutes to 2 hours, preferably 1 hour. The conditions of heat and pressure which are thus created within the closed chamber ensure that the feedstock is rendered biologically sterile, and furthermore solid organic matter within the feedstock, particularly bones, becomes soft and pliable under these conditions. When the pressure is released from the chamber, the essentially organic component of the feedstock including bones which have become soft and pliable are forced outwardly through the perforations of the container leaving solid inorganic matter such as bottles, cans and the like behind. The organic matter is thus separated from the inorganic matter. If desired, the solid inorganic matter may be further processed for recovery and recycling of any ferrous and non-ferrous metal or plastics or glass therein.

As the organic matter of the feedstock is forced through the perforations as described above, the material is shredded and broken up. If desired, one or more enzymes, acids or alkalis may be added to the feedstock to assist in hydrolysis of cellulose and other components thereof prior to the alcohol production step. The feedstock is then fermented for example by means of yeasts or bacteria in the known manner to form an ethanol-containing fermentation mash, and carbon dioxide produced during the fermentation may, if desired, be recovered and stored for later use as required. After fermentation, the fermentation mash may be separated, preferably by passage to a centrifuge where the solid fraction is separated from the liquid fraction thereof. The solid fraction retains high nutritional value and is preferably recovered for use as an animal foodstuff. Ethanol may be recovered from the liquid fraction by known means, preferably by distillation, and the balance of the liquid fraction than passed for further processing.

If desired, after recovery of the ethanol, the balance of the liquid fraction may be passed to a centrifuge for recovery of fats prior to the product being passed to an anaerobic digester. In this digester, "bio-gas" is produced in the known manner and subsequently separated into methane and carbon dioxide.

If desired, the effluent from the anaerobic digester may be further processed for removal of the remaining nutrients therein as described in more detail below.

It will be appreciated from the foregoing description that an important aspect of the present invention resides in the fermentation of the organic matter of the feedstock, for example for the production of ethanol. This production of ethanol may be enhanced by hydrolysis of polysaccharide materials prior to fermentation and, as described above, this may be achieved by addition of an enzyme or an acid such as hydrochloric acid to the feedstock prior to fermentation. Such acids may be subsequently neutralized with basic materials such as lime to form salts which are of benefit, for example in animal feedstuffs.

In another aspect of the present invention, there is provided apparatus for treatment of a waste material feedstock for the separation and recovery of organic and inorganic matter therein, said apparatus comprising:—

(a) a closed chamber;

(b) means for feeding said waste material feedstock into said chamber;

(c) means for subjecting said waste material feedstock in said chamber to heat under pressure to sterilise the feedstock and soften solid organic matter therein;

(d) means for suddenly releasing the pressure from said chamber; and

(e) fermentation means wherein the organic matter of said sterilised feedstock can be fermented to produce a fermentation mash; characterised in that:

(f) a container having perforations therein for separating solid inorganic matter from said sterilized feedstock, is mounted within said closed chamber in spaced relation to the inner surface of said closed chamber said feeding means being arranged to feed to said waste material feedstock into said container; and

(g) means for agitating said waste material feedstock while in said container is provided.

Where an ethanol-containing fermentation mash is produced the apparatus may further comprise:—

(h) means to separate the fermentation mash into a solid fraction and a liquid fraction;

(i) distillation means for recovery of ethanol from said liquid fraction; and, if desired,

(j) anaerobic digester means wherein the residue from said distillation means can be digested for the recovery of methane.

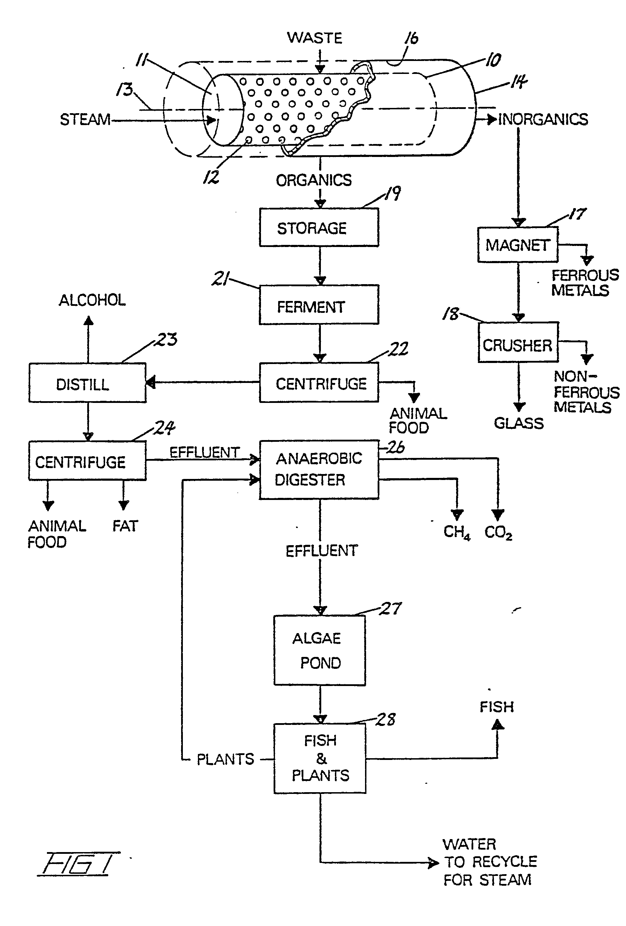

Apparatus embodying features of the present invention and which may be employed to carry out a preferred embodiment of the improved process of this invention is shown schematically in Figure 1 of the accompanying drawing.

Referring now to the drawing for a better understanding of the present invention, there is shown waste material, such as industrial, commercial, agricultural or household garbage being fed into a perforated drum or container 10 having closed ends 11 and perforations 12 at the sides thereof. In actual practice, it has been found that a container 10 cylindrical in shape and having 1.9 cm (3/4 ) inch perforations at its sides is satisfactory. The container 10 is shown as being mounted for rotation about its longitudinal axis 13 inwardly of a closed chamber 14 and in spaced relation to the inner surface 16 thereof as shown. In an alternative construction, however, the container 10 may be of inverted conical shape and mounted for rotation abouts its vertical axis.

After the waste material is fed into the container 10, it is agitated by rotation of the container and subjected to heat and pressure by suitable means, such as by introducing steam into the closed chamber 14 for a period ranging from twenty minutes to two hours to bring the pressure therein up to a pressure ranging from 1.38 to 6.89 bar (20 to 100 psi). The waste material is thus biologically sterilized and the organic matter, such as vegetable matter, bones, meat scraps, paper and the like, are softened and partially hydrolyzed. In actual practice, it has been found that when the steam is introduced into the chamber 14 at a pressure of 4.14 bar (60 psi) for approximately one hour, the process operates satisfactorily in every respect with household garbage and sewage and food scraps from restaurants. During the steam injection period, the container 10 is agitated, such as by rotating it about its longitudinal axis 13 within the chamber 14 by a conventional drive unit, not shown.

After the waste material is sterilized and the organic matter softened, the pressure within the chamber 14 is released suddenly whereby the organic matter is separated from the inorganic matter such as bottles, cans, ceramics and the like. This is accomplished due to the sudden depressurization within the chamber 14 which causes the softened organic matter to be forced out of the container 10 through the perforations 12 with only solid inorganic matter being left therein.

The solid inorganic matter is removed from the container 10 by suitable means for further processing and separation into useful glass, ferrous and non-ferrous materials. This may be accomplished by passing the solids over a magnet 17 to remove the ferrous metals and then introducing the remainder thereof to a conventional crusher 18 where the glass is shattered and recovered from non-ferrous metals for reuse in a manner well understood in the art.

The softened organic matter is shredded and broken up as it is forced through the perforations 12 and is then transferred to a conventional storage container 19 where suitable additives to hydrolyse the organic matter, such as acids, alkali or enzymes, may be added. The shredded and treated organic matter is transferred from the storage container 19 to a fermentation chamber 21 where it is fermented with yeast or bacteria in a conventional manner to form an ethanol-containing mash.

The mash is separated into liquid and solid portions by passing it through a conventional centrifuge 22. This solid portion contains high nutritional value and is preferably recovered for use as an animal feed supplement. This may be accomplished by feeding in a wet state to form a wet feed or by drying the solid portion in a conventional manner to a moisture content not greater than 13% by weight. After drying, the solid portion is passed through a conventional milling apparatus such as a hammer mill where it is pulverized into a meal or granular type substance with high protein content.

The separated liquid portion of the mash is passed to a distillation column 23 for the recovery of ethanol, with the residue therefrom being transferred to a second centrifuge 24 for the recovery of fats and animal feed before passing the solubles into an anaerobic digester 26. To aid in the production of ethanol, acid, such as hydrochloric acid, alkali or enzymes, such as cellulases may be added to the organic matter prior to fermentation. This will enhance the hydrolysis of the polysaccharide materials contained therein. In the anaerobic digester 26, a "bio-gas" is produced and separated into carbon dioxide and methane in a conventional scrubber, not shown. In view of the fact that recovering methane, carbon dioxide and ethanol from the processes mentioned above is well known in the art, no further description is deemed necessary.

The effluent from the anaerobic digester 26 may be transferred to an algae pond 27 where algae is grown from the remaining nutrients through photosynthesis. The algae may be harvested and used as an animal feedstuff or contents of the algae pond 27 may be passed into a fish pond 28 where a portion of the algae is used primarily as a food for fish with the remainder of the pond contents being used as food for water plants. The plants may be harvested and again, either used as an animal feedstuff or returned to the anaerobic digester 26, while at the same time the water is being cleaned and recycled for use in steam production.

From the foregoing, the operation of the preferred embodiment of the improved process for treating waste material for the recovery of useful organic and inorganic matter will be readily understood.

As an illustration of the effectiveness of the process of this invention, it is noted that in a pilot plant operation, amounts of more than 224 litres of anhydrous ethanol have been recovered per ton (dry weight basis) of municipal waste (50 gallons per ton).

It will thus be seen that this invention provides an improved process for treating waste material which is simple and very economical to carry out. Also this improved process permits waste material to be diverted from landfills and overloaded sewage systems and converted into useful and valuable fuels and supplements for animal feed.

**Claims**

1. A process for treating a waste material feedstock for the separation and recovery of organic and inorganic matter contained therein, which comprises the steps of:—

(a) feeding said waste material feedstock into a closed chamber;

(b) subjecting said waste material feedstock in said chamber to heat under pressure ranging preferably from 1.38 to 6.89 bar (20 to 100 p.s.i.) for a period ranging preferably from twenty minutes to two hours to sterilize said waste material feedstock and soften said organic matter contained therein;

(c) releasing the pressure in said chamber suddenly; and

(d) subjecting the organic component of said sterilised feedstock to fermentation to form a fermentation mash;

characterised in that:

(e) said waste material feedstock is fed into a container having perforations therein mounted within said closed chamber in spaced relation to the inner surface of said closed chamber;

(f) said waste material feedstock is agitated while in said container before said sudden release of pressure;

(g) during said sudden release of pressure, the softened organic matter is forced outwardly of said container through said perforations, so that a softened essentially organic component of the sterilised feedstock is separated from solid inorganic matter in said container; and

(h) said separated softened essentially organic component is then subjected to said fermentation step.

2. The process of claim 1, wherein steam under pressure is introduced into the chamber to heat and pressurize said waste material in said container.

3. The process of claim 2 wherein the steam is introduced for a period of approximately 1 hour at a pressure of approximately 4.14 bar (60 psi).

4. The process of any one of claims 1 to 3, wherein said separated solid inorganic matter is further separated into ferrous and non-ferrous materials by subjecting it to a magnetic field to remove said ferrous material therefrom.

5. The process of any one of claims 1 to 4, wherein said separated softened essentially organic component is subjected to fermentation by yeast to form said mash.

6. The process of any one of claims 1 to 4, wherein said separated softened essentially organic component is subjected to fermentation by bacteria to form said mash.

7. The process of any one of claims 1 to 6, wherein said fermentation mash is separated into a liquid portion, which is treated for the recovery of fuels, and a solid portion, which is treated for the recovery of animal feed supplements.

8. The process of claim 7, wherein said liquid portion is distilled for the recovery of ethanol with the residue thereof being fed into an anaerobic digester for the recovery of methane.

9. The process of claim 7 or claim 8, wherein said solid portion is dried to a maximum moisture content of approximately 13% by weight.

10. The process of any one of claims 1 to 9, wherein said essentially organic component of said sterilized feedstock is subjected to hydrolysis prior to said fermentation step.

11. The process of claim 10, wherein hydrolysis is performed by addition of at least one enzyme, acid or alkali to said essentially organic component.

12. Apparatus for treatment of a waste material feedstock for the separation and recovery of organic and inorganic matter therein, said apparatus comprising:

(a) a closed chamber (14);

(b) means for feeding said waste material feedstock into said chamber (14);

(c) means for subjecting said waste material feedstock in said chamber (14) to heat under pressure to sterilise the feedstock and soften solid organic matter therein;

(d) means for suddenly releasing the pressure from said chamber; and

(e) fermentation means (21) wherein the organic matter of said sterilised feedstock can be fermented to produce a fermentation mash;

characterised in that:

(f) a container (10) having perforations (12) therein for separating solid inorganic matter from said sterilized feedstock, is mounted within said closed chamber (14) in spaced relation to the inner surface of said closed chamber (14), said feeding means being arranged to feed said waste material feedstock into said container (10); and

(g) means for agitating said waste material feedstock while in said container (10) is provided.

13. The apparatus of claims 12, further comprising:

(h) means (22) to separate said fermentation mash into a solid fraction and a liquid fraction;

(i) distillation means (23) for recovery of ethanol from said liquid fraction; and, if desired,

(j) anaerobic digester means (26) wherein the residue from said distillation means (23) can be digested for the recovery of methane.

**Patentansprüche**

1. Verfahren zur Behandlung eines Abfall-Ausgangsmaterials zum Abscheiden und Wiedergewinnen darin enthaltener organischer und anorganischer Substanzen,

bestehend aus den Verfahrensschritten:

(a) Zuführen des Abfall-Ausgangsmaterials in eine geschlossene Kammer;

(b) Aussetzen des Abfall-Ausgangsmaterials in der Kammer einer Erhitzung unter einem Druck, der vorzugsweise von 1,38 bis 6,89 bar (20 bis 100 p.s.i.) reicht, während einer Zeitdauer, die vorzugsweise von 20 Minuten bis 2 Stunden reicht, um das Abfall-Ausgangsmaterial zu sterilisieren und die darin enthaltene organische Substanz zu erweichen;

(c) plötzliches Entlasten des Druckes in der Kammer; und

(d) Unterziehen des organischen Bestandteils des sterilisierten Ausgangsmaterials einer Fermentation zur Bildung eines Fermentationsbreies;

dadurch gekennzeichnet, daß

(e) das Abfall-Ausgangsmaterial in einen Lochungen aufweisenden Behälter eingeführt wird, der innerhalb der geschlossenen Kammer in beabstandeter Zuordnung zu der Innenfläche der geschlossenen Kammer angeordnet ist;

(f) das Abfall-Ausgangsmaterial bewegt wird, während es sich in dem Behälter vor der plötzlichen Druckenlastung befindet;

(g) während der plötzlichen Druckentlastung die erweichte organische Substanz aus dem Behälter durch die Lochungen nach außen gedrückt wird, so daß ein erweichter, im wesentlichen organischer Bestandteil des sterilisierten Ausgangsmaterials von fester anorganischer Substanz in dem Behälter abgeschieden wird, und

(h) der abgeschiedenen erweichte, im wesentlichen organische Bestandteil, dann dem Fermentationsschritt unterworfen wird.

2. Verfahren nach Anspruch 1, dadurch gekennzeichnet, daß Dampf unter Druck in die Kammer zur Erhitzung und zum unter Druck setzen des Abfallmaterials in dem Behälter eingeführt wird.

3. Verfahren nach Anspruch 2, dadurch gekennzeichnet, daß der Dampf für eine Zeitdauer von annähernd einer Stunde mit einem Druck von annähernd 4,14 bar (60 psi) eingeführt wird.

4. Verfahren nach einem der Ansprüche 1 bis 3, dadurch gekennzeichnet, daß die abgeschiedene feste anorganische Substanz weiter in eisenhaltige und nicht eisenhaltige Materialien getrennt wird, indem sie einem magnetischen Feld ausgesetzt wird, um daraus das eisenhaltige Material zu entfernen.

5. Verfahren nach einem der Ansprüche 1 bis 4, dadurch gekennzeichnet, daß der angeschiedene erweichte, im wesentlichen organische Bestandteil einer Fermentation durch Hefe zur Bildung des Breies unterworfen wird.

6. Verfahren nach einem der Ansprüche 1 bis 4, dadurch gekennzeichnet, daß der abgeschiedene erweichte, im wesentlichen organische Bestandteil einer Fermentation durch Bakterien zur Bildung des Breies unterworfen wird.

7. Verfahren nach einem der Ansprüche 1 bis 6, dadurch gekennzeichnet, daß der Fermentationsbrei in einen flüssigen Anteil, der zur Wiedergewinnung von Brennstoffen behandelt wird, und in einen festen Bestandteil getrennt wird, der zur Zurückgewinnung von Tierfutterzusätzen behandelt wird.

8. Verfahren nach Anspruch 7, dadurch gekennzeichnet, daß der flüssige Anteil zur Zurückgewinnung von Äthanol destilliert wird, wobei dessen Rest in einen anaerobischen Digester zur Rückgewinnung von Methan eingeführt wird.

9. Verfahren nach Anspruch 7 oder 8, dadurch gekennzeichnet, daß der feste Anteil bis auf einen maximalen Feuchtigkeitsgehalt von etwa 13 Gewichtprozent getrocknet wird.

10. Verfahren nach einem der Ansprüche 1 bis 9, dadurch gekennzeichnet, daß der im wesentlichen organische Bestandteil des sterilisierten Ausgangsmaterials vor dem Fermentationsschritt einer Hydrolyse unterworfen wird.

11. Verfahren nach Anspruch 10, dadurch gekennzeichnet, daß die Hydrolyse durch Zusatz von wenigstens einem Enzym, einer Säure oder einem Alkali zu dem im wesentlichen organischen Bestandteil durchgeführt wird.

12. Vorrichtung zur Behandlung eines Abfall-Ausgangsmaterials zur Abscheidung und zur Wiedergewinnung von darin enthaltener organischer und anorganischer Substanz, wobei die Vorrichtung sich zusammensetzt aus:

(a) einer geschlossenen Kammer (14);

(b) einer Einrichtung zur Zuführung des Abfall-Ausgangsmaterials in die Kammer (14);

(c) einer Einrichtung zum Aussetzen des Abfall-Ausgangsmaterials in der Kammer (14), einer Erhitzung unter Druck zum Serilisieren des Ausgangsmaterials und zum Erweichen darin enthaltener fester organischer Substanz;

(d) einer Einrichtung zum plötzlichen Entlasten des Druckes aus der Kammer; und

(e) einer Fermentationseinrichtung (21), in der die organische Substanz des sterilisierten Ausgangsmaterials zur Erzeugung eines Fermentationsbreies fermentiert werden kann;

dadurch gekennzeichnet, daß

(f) ein Behälter (10), in dem Lochungen (12) zum Abscheiden fester anorganischer Substanz von dem sterilisierten Ausgangsmaterial vorgesehen sind, innerhalb der geschlossenen Kammer (14) in beabstandeter Zuordnung zu der Innenfläche der geschlossenen Kammer (14) montiert ist, wobei die Zuführeinrichtung so angeordnet ist, daß sie das Abfall-Ausgangsmaterial in den Behälter (10) einführt; und

(g) eine Einrichtung zum Bewegen des in dem Behälter (10) befindlichen Abfall-Ausgangsmaterials vorgesehen ist.

13. Vorrichtung nach Anspruch 12, weiterhin gekennzeichnet durch:

(h) eine Einrichtung (22) zum Trennen des Fermentationsbreies in eine feste Fraktion und in eine flüssige Fraktion;

(i) eine Destillationseinrichtung (23) zur Rückgewinnung von Äthanol aus der flüssigen Fraktion; und bei Bedarf;

(j) eine anaerobische Digestereinrichtung (26), in der der Rest aus der Destillationseinrichtung (23) zur Wiedergewinnung von Methan digestiert werden kann.

**Revendications**

1. Procédé pour le traitement d'une charge d'alimentation de matière de déchets pour la séparation et la récupération de la matière organique et minérale qui est contenue, quie comprend les étapes consistant à:

(a) introduire ladite charge d'alimentation de matière de déchets dans une chambre fermée;

(b) soumettre cette charge d'alimentation de matière de déchets dans ladite chambre à de la chaleur sous une pression allant de préférence de 1,38 à 6,89 × 10⁵ Pa pendant une durée allant de préférence de 20 minutes à deux heures pour stériliser ladite charge d'alimentation de matière de déchets et ramollir la matière organique qui y est contenue;

(c) relâcher la pression brusquement dans ladite chambre; et

(d) soumettre le constituant organique de ladite charge d'alimentation stérilisée à une fermentation pour former une bouillie de fermentation;

caractérisé par le fait que:

(e) ladite charge d'alimentation de matière de déchets est introduite dans un récipient dans lequel se trouvent des perforations monté dans ladite chambre fermée, à distance de la surface interne de ladite chambre fermée;

(f) ladite charge d'alimentation de matière de déchets est agitée tandis qu'elle se trouve dans ledit récipient avant ledit relachement brusque de la pression;

(g) au cours dudit relachement brusque de la pression, la matière organique remollie est forcée vers l'extérieur dudit récipient à travers lesdites perforations, de telle sorte qu'un constituant essentiellement organique ramolli de la charge d'alimentation stérilisée est séparé de la matière minérale solide présente dans ledit récipient; et

(h) ledit constituant essentiellement organique ramolli séparé est alors soumis à ladite étape de fermentation.

2. Procédé selon la revendication 1, caractérisé par le fait que de la vapeur sous pression est introduite dans la chambre pour chauffer et mettre sous pression ladite matière de déchets dans ledit récipient.

3. Procédé selon la revendication 2, caractérisé par le fait que de la vapeur est introduite pendant une durée d'environ une heure sous une pression d'environ $4,14 \times 10^5$ Pa.

4. Procédé selon l'une quelconque des revendications 1 à 3, caractérisé par le fait que ladite matière minérale solide séparée est encore séparée en des matières ferreuses et non-ferreuses en la soumettant à un champ magnétique pour éliminer ladite matière ferreuse de celle-ci.

5. Procédé selon l'une quelconque des revendications 1 à 4, caractérisé par le fait que ledit constituant essentiellement organique ramolli séparé est soumis à une fermentation par une levure pour former ladite bouillie.

6. Procédé selon l'une quelconque des revendications 1 à 4, caractérisé par le fait que ledit constituant essentiellement organique ramolli séparé est soumis à une fermentation par des bactéries pour former ladite bouillie.

7. Procédé selon l'une quelconque des revendications 1 à 6, caractérisé par le fait que ladite bouillie de fermentation est séparée en une portion liquide, qui est traitée pour la récupération de combustibles, et en une portion solide qui est traitée pour la récupération de suppléments pour l'alimentation animale.

8. Procédé selon la revendication 7, caractérisé par le fait que ladite portion liquide est distillée pour la récupération de l'éthanol, le résidu de celle-ci étant introduit dans un digesteur anaèrobie pour la récupération de méthane.

9. Procédé selon la revendication 7 ou 8, caractérisé par le fait que ladite portion solide est séchée à une teneur en humidité maxima d'environ 13 % en poids.

10. Procédé selon l'une quelconque des revendications 1 à 9, caractérisé par le fait ledit constituant essentiellement organique de ladite charge d'alimentation stérilisée est soumis à une hydrolyse avant ladite étape de fermentation.

11. Procédé selon la revendication 10, caractérisé par le fait que l'hydrolyse est effectuée par addition d'au moins une enzyme, un acide ou un alcali, audit constituant essentiellement organique.

12. Appareil pour la traitement d'une charge d'alimentation de matière de déchets pour la séparation et la récupération de la matière organique et minérale qui s'y trouve, ledit appareil comprenant:

(a) une chambre fermée (14);

(b) des moyens pour introduire ladite charge d'alimentation de matière de déchets dans ladite chambre (14);

(c) des moyens pour soumettre ladite charge d'alimentation de matière de déchets dans ladite chambre (14) à la chaleur sous pression pour stériliser la charge d'alimentation et ramollir ladite matière organique solide qu'elle contient;

(d) des moyens pour relâcher brusquement la pression dans ladite chambre; et

(e) des moyens de fermentation (21) dans lesquels la matière organique de ladite charge charge d'alimentation stérilisée peut être fermentée pour produire une bouillie de fermentation;

caractérisé par le fait que

(f) un récipient (10) comportant des perforations (12) pour séparer la matière minérale solide de ladite charge d'alimentation stérilisée est monté dans ladite chambre fermée (14) à distance de la surface interne de ladite chambre fermée (14), ledit moyen d'alimentation étant conçu pour introduire ladite charge d'alimentation de matière de déchets dans ledit récipient (10); et

(g) des moyens sont prévus pour agiter ladite charge d'alimentation de matière de déchets alors qu'elle se trouve dans ledit récipient (10).

13. Appareil selon la revendication 12, comprenant en outre:

(h) des moyens (22) pour séparer la bouillie de fermentation en une fraction solide et en une fraction liquide;

(i) des moyens de distillation (23) pour récupérer l'éthanol à partir de ladite fraction liquide; et, si on le désire,

(j) des moyens de digestion anaérobie (26) dans lesquels le résidu provenant desdits moyens de distillation (23) peut être digéré pour la récupération du méthane.

FIG 1